Europäisches Patentamt

European Patent Office

Office européen des brevets

(19)

(11) Veröffentlichungsnummer: **0 376 098**

**A2**

(12) **EUROPÄISCHE PATENTANMELDUNG**

(21) Anmeldenummer: 89123314.0

(22) Anmeldetag: 16.12.89

(51) Int. Cl.5: **G01N 33/00**

(30) Priorität: 24.12.88 DE 3843920

(43) Veröffentlichungstag der Anmeldung:
04.07.90 Patentblatt 90/27

(84) Benannte Vertragsstaaten:
**BE DE FR GB NL**

(71) Anmelder: **Drägerwerk Aktiengesellschaft
Moislinger Allee 53-55
D-2400 Lübeck 1(DE)**

(72) Erfinder: **Jessel, Wolfgang, Dr.
Eichenweg 17
D-2067 Reinfeld(DE)**
Erfinder: **Masurat, Kurt
Kiefernweg 18
D-2401 Hamberge(DE)**

(54) Prüfbehälter für Gasmess- und Gasnachweisgeräte.

(57) Ein Prüfbehälter für die Überprüfung von Gasmeß- und Gasnachweisgeräten, in welchem eine Prüfsubstanzatmosphäre einer bestimmten Konzentration an Prüfsubstanz vorliegt, in die der Meßkopf des Gasmeßgerätes einbringbar ist, soll so verbessert werden, daß in ihm eine solche Prüfsubstanzatmosphäre auch über einen längeren Zeitraum bereitgestellt werden kann, die sich in ihrer Zusammensetzung nur unwesentlich ändert und nicht erst vor Durchführung einer Überprüfung hergestellt werden muß. Dabei soll der Behälter leicht herstellbar, von geringem Gewicht, leicht handzuhaben und bequem transportierbar und lagerfähig sein. Dazu ist vorgesehen, in dem mit einer durchbrechbaren Versiegelung (5) gasdicht abgeschlossenen Prüfbehälter (1) eine Flüssigkeit (7) einzubringen, die aus einer Lösung der Prüfsubstanz in einem für den Meßkopf inerten Lösungsmittel von solcher Menge und Konzentration zusammengesetzt ist, daß ein an der Prüfsubstanz gesättigter Dampf vorliegt.

## Prüfgasbehälter für Gasmeß- und Gasnachweisgeräte

Die Erfindung betrifft einen Prüfbehälter für die Überprüfung von Gasmeß- und Gasnachweisgeräten, in welchem eine Prüfsubstanzatmosphäre einer bestimmten Konzentration an Prüfsubstanz vorliegt, in die der Meßkopf des Gasmeßgerätes einbringbar ist.

Ein derartiger Prüfbehälter ist in der DE-Z: Dräger-Heft 303 (1976) Seite 15 beschrieben. Der bekannte Prüfbehälter dient zur Kalibrierung von Gasmeßgeräten, wie z.B. Geräte zur Bestimmung von Schwefelwasserstoff. Zur Erzeugung einer Prüfsubstanzatmosphäre werden in einen Becher Prüfgasampullen eingebracht, welche vor Benutzung des Prüfbehälters in diesem mechanisch zerstört werden, so daß sich der Ampulleninhalt in dem Bechervolumen verteilt. Durch eine Öffnung im Prüfbehälter wird der Meßkopf des zu kalibrierenden Prüfgerätes gesteckt, und die entsprechende Konzentration an Schwefelwasserstoff soll bei kalibriertem Gerät angezeigt werden.

Bei dem bekannten Prüfbehälter ist es von Nachteil, daß die Prüfampullen zuvor mit äußerster Sorgfalt hergestellt bzw. das Prüfgas gemischt und in die Ampullen abgefüllt werden müssen. Das Prüfgas in den Ampullen kann sich an der Glasinnenwand absetzen und somit die Konzentration des Prüfgases verfälschen. Außerdem ist es umständlich, die Glasampullen in dem Behälter selbst erst zerbrechen zu müssen, nachdem der Meßkopf eingeführt wurde. Die nicht verschließbare Öffnung des Prüfbehälters führt zu einer Vermengung der Prüfatmosphäre des Behälters mit der Umgebungsluft, sofern eine längere Zeitspanne vergeht, bis der Meßkopf in den Behälter eingetaucht wird. Außerdem ist eine innige Vermischung von Dämpfen, die schwerer sind als Luft, mit der im Behälter befindlichen Luft nicht in jedem Falle sichergestellt, so daß nicht verhindert werden kann, daß der Meßkopf mehr oder weniger zufällig in einen solchen Behälterbereich kommt, in welchem die gewünschte Prüfgasatmosphäre noch nicht vorliegt oder durchmischt ist.

Der vorliegenden Erfindung liegt somit die Aufgabe zugrunde, einen Prüfbehälter der genannten Art so zu verbessern, daß in ihm eine solche Prüfsubstanzatmosphäre auch über einen längeren Zeitraum bereitgestellt werden kann, die sich in ihrer Zusammensetzung nur unwesentlich verändert und nicht erst vor Durchführung einer Überprüfung hergestellt werden muß. Dabei sollen die Behälter leicht herstellbar, von geringem Gewicht, leicht handzuhaben und bequem transportierbar und lagerfähig sein.

Die Lösung der Aufgabe erfolgt dadurch, daß in den mit einer durchbrechbaren Versiegelung gasdicht abgeschlossenen Prüfbehälter eine Flüssigkeit eingebracht ist, die aus einer Lösung der Prüfsubstanz in einem für den Meßkopf inerten Lösungsmittel von solcher Menge und Konzentration zusammengesetzt ist, daß ein an der Prüfsubstanz gesättigter Dampf vorliegt.

Der Vorteil der Erfindung liegt im wesentlichen darin, daß durch definierte Präparation der Lösung die Prüfsubstanz innerhalb des Behälters in einer solchen Menge vorliegt, daß sich ein stabiler, an der Prüfsubstanz gesättigter Dampf bilden kann. Auch bei möglichen Kondensationen dieses Dampfes an den Behälterwänden verbleibt genügend Flüssigkeit übrig, daß die Kondensationsverluste ausgeglichen werden können. Die Herstellung eines Lösungsgemisches ist einfacher als die Herstellung einer definierten gasförmigen Prüfsubstanz vorgegebener Konzentration.

Bei einem typischen Behältervolumen von etwa 100 mL reicht eine Flüssigkeitsmenge von 10 bis 20 mL in der Regel aus. Der versiegelte Prüfbehälter kann mit seiner flüssigen Prüfsubstanz über einen längeren Zeitraum gelagert werden, wobei in einfacher Weise vor Durchführung der Prüfung kontrolliert werden kann, Ob in ihm noch genügend Flüssigkeit vorhanden ist: er braucht dazu lediglich hin- und hergeschüttelt zu werden. Damit können leicht solche Prüfbehälter ausgesondert werden, in denen die benötigte Prüfsubstanzatmosphäre nicht mehr sichergestellt ist. Die Prüfsubstanz kann einerseits eine von dem Meßkopf nachzuweisende Substanz in Lösung sein, z. B. gelöstes Toluol für einen Meßkopf zur Bestimmung von Toluol in Luft, sie kann aber andererseits auch eine Alkoholwasserlösung sein, in welcher Alkohol von bestimmter Konzentration vorliegt, die in Dampfform ausgewählte Prozentwerte einer unteren Explosionsgrenze für Nachweisgeräte explosibler Gasgemische festlegen. In ersterem Falle hat man einen einfachen Prüfbehälter, mit dem sogar in gewissen Grenzen Kalibrierungen eines Gasmeßgerätes durchgeführt werden können, im letzteren Falle stehen einfach herstellbare und handhabbare Konzentrationen prozentualer unterer Explosionsgrenzen mit ein- und demselben Prüfsubstanzgemisch zur Verfügung.

Die Versiegelung wird am besten so ausgeführt, daß sie von dem Meßkopf durchbrechbar ist, indem beispielsweise mittels eines Dornes oder eines gerändelten Meßkopfrandes die Versiegelung in dem Umfange des Meßkopfes durchstoßen wird. Der übrige noch verbleibende Randbereich der durchbrochenen Versiegelung dient dann gleichzeitig als Abschirmung der Dampfatmosphäre im Behälter gegenüber der Umgebungsluft.

2

Um einen Austausch der Dampfatmosphäre zur Umgebung noch weiter zu unterdrücken, ist es zweckmäßig, eine Aufnahme zur dampfdichten Anbringung des Meßkopfes in die durchbrochene Versiegelung vorzusehen. Dies kann beispielsweise dadurch geschehen, daß der Rand der Behälteröffnung zweckmäßigerweise soweit verengt wird, und mit einem Dichtbelag ausgekleidet wird, daß die Konturen des Meßkopfes von ihm aufgenommen werden.

Da die Ausbildung einer in der gesättigten Dampfatmosphäre vorliegenden Konzentration der Prüfsubstanz unter anderem auch von der Umgebungstemperatur abhängig ist, ist es günstig, an den Behälter einen Indikator für die Behältertemperatur anzubringen. Derartige Indikatoren sind als selbstklebende Bänder erhältlich, auf denen mikroverkapselte Flüssigkristalle aufgebracht sind, die je nach Temperatur eine Verfärbung zeigen. Anhand von Temperaturdampfdruckkurven kann somit auf einfache Weise der bei der entsprechenden Temperatur vorliegende Sättigungsdampfdruck und damit auch die Konzentration in der Prüfsubstanzatmosphäre ermittelt werden.

Um den Behälter vor Temperaturschwankungen weitgehend zu schützen, ist es vorteilhaft, ihn mit einem wärmeisolierenden Überzug zu versehen. In diesem Überzug sollte zur Sichtbarmachung eines Temperaturindikators ein entsprechendes Fenster vorgesehen sein.

Eine besonders preisgünstige Ausführungsform des Prüfbehälters wird dadurch gebildet, daß er aus einem Styroporbecher mit einer Aluminiumfolienversiegelung über der Becheröffnung besteht. Diese Becher sind billig herstellbar, leicht zu transportieren und zu lagern und sind als ideale Einmalartikel anzusehen.

Um eine mögliche Diffusion bestimmter dampfförmiger Substanzen aus dem Becher zu verhindern bzw. um den Prüfbehälter gegenüber solchen Substanzen beständig zu machen, die das Behältermaterial anlösen könnten, ist es zweckmäßig, einen Plastbehälter mit einer Innenkaschierung aus Aluminium zu versehen.

Ein Ausführungsbeispiel der Erfindung wird anhand der schematischen Zeichnung dargestellt und im folgenden näher erläutert.

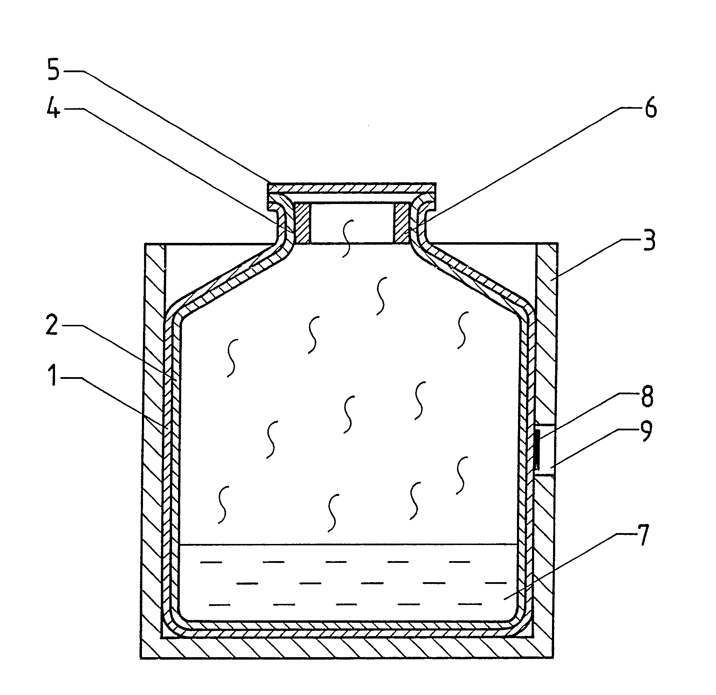

In der einzigen Figur ist ein Prüfbehälter in Form eines Bechers (1) dargestellt, dessen Innenraum mit einer Aluminiumbeschichtung (2) ausgekleidet und in einem becherförmigen Überzug (3) zur Wärmeisolierung aufgenommen ist. Die Becheröffnung (4) ist mit einer Versiegelung (5) aus einer Alumiumfolie verschlossen, welche mit der über den Becherrand hinausgezogenen Innenbeschichtung (2) zu einer Einheit verschweißt ist. Die Innenseite der Becheröffnung (4) trägt weiterhin eine Aufnahme (6), in welche ein nicht dargestellter Meßkopf eines ebenfalls nicht dargestellten Meß- bzw. Nachweisgerätes zur Überprüfung eingesteckt werden kann. Auf dem Becherboden befindet sich die Prüfsubstanz (7) als Flüssigkeit, welche den Innenraum des Bechers (1) als Dampf füllt. Auf der Außenwand des Bechers (1) ist an einer geeigneten Stelle ein plakettenförmiger Temperaturindikator (8) aufgeklebt, welcher durch ein Fenster (9) in dem Überzug (3) von außen einsehbar ist.

## Ansprüche

1. Prüfbehälter für die Überprüfung von Gasmeß- und Gasnachweisgeräten, in welchem eine Prüfsubstanzatmosphäre einer bestimmten Konzentration an Prüfsubstanz vorliegt, in die der Meßkopf des Gasmeßgerätes einbringbar ist, dadurch gekennzeichnet, daß in den mit einer durchbrechbaren Versiegelung (5) gasdicht abgeschlossenen Prüfbehälter eine Flüssigkeit (7) eingebracht ist, die aus einer Lösung der Prüfsubstanz in einem für den Meßkopf inerten Lösungsmittel von solcher Menge und Konzentration zusammengesetzt ist, daß ein an der Prüfsubstanz gesättigter Dampf vorliegt.

2. Prüfbehälter nach Anspruch 1, dadurch gekennzeichnet, daß die Prüfsubstanz das gelöste, in die Dampfphase übergehende nachzuweisende Gas enthält.

3. Prüfbehälter nach Anspruch 1, dadurch gekennzeichnet, daß die Prüfsubstanz ein von dem nachzuweisenden unterschiedliches gelöstes, in die Dampfphase übergehendes Gas enthält.

4. Prüfbehälter nach einem der Ansprüche 1 bis 3, dadurch gekennzeichnet, daß die Versiegelung (5) von dem Meßkopf durchbrechbar ist.

5. Prüfbehälter nach einem der Ansprüche 1 bis 4, dadurch gekennzeichnet, daß der Behälter (1) eine Aufnahme (6) zur dampfdichten Anbringung des Meßkopfes in die durchbrochene Versiegelung (5) aufweist.

6. Prüfbehälter nach einem der Ansprüche 1 bis 5, dadurch gekennzeichnet, daß der Behälter (1) mit einem die Behältertemperatur anzeigenden Indikator (8) versehen ist.

7. Prüfbehälter nach einem der Ansprüche 1 bis 6, dadurch gekennzeichnet, daß der Behälter (1) mit einem wärmeisolierenden Überzug (3) ausgestattet ist.

8. Prüfbehälter nach Anspruch 7, dadurch gekennzeichnet, daß der Überzug (3) ein Sichtfenster (9) für den Indikator (8) enthält.

9. Prüfbehälter nach einem der Ansprüche 1 bis 8, dadurch gekennzeichnet, daß der Behälter aus einem Polystyrolschaumbecher (1) mit einer Aluminiumfolienversiegelung (5) über der Becheröffnung (4) besteht.

10. Prüfbehälter nach einem der Ansprüche 1 bis 9, dadurch gekennzeichnet, daß der Behälter aus einem aluminiuminnenbeschichteten Plastbecher (1) mit einer Aluminiumfolienversiegelung (5) über der Becheröffnung (4) besteht.